# EUROPEAN PATENT APPLICATION

(11) **EP 2 942 079 A1**
(43) Date of publication of application: **11.11.2015**
(21) Application number: 15163184.3
(22) Date of filing: 10.04.2015
(51) Int. Cl.: A61M 25/10

(54) **Balloon catheter**

(30) Priority: 10.05.2014 JP 2014098193
(71) Applicant: ASAHI INTECC CO., LTD., Nagoya-shi, Aichi 463-0024 (JP)
(72) Inventor: Nakagawa, Yuta c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(57) **Abstract**

A balloon catheter (10) including an inner tube (50) and a balloon (20) secured to the inner tube, in which at least one rough portion (80, 80a, 80b, 80c) that has a large surface roughness and at least one smooth portion (90, 90a, 90b) that has a surface roughness that is smaller than the surface roughness of the rough portion are formed on a surface of the balloon (20), and the at least one rough portion (80, 80a, 80b, 80c) and the at least one smooth portion (90, 90a, 90b) are configured so that the rough portion (80, 80a, 80b, 80c) is positioned between the inner tube (50) and the smooth portion (90, 90a, 90b) in a cross sectional view of the balloon catheter (10), when the balloon (20, 20a, 20b) is folded around the outer periphery of the inner tube (50).

## Description

### BACKGROUND OF THE INVENTION

### 1. Field of the Invention

The present invention relates to a balloon catheter that is inserted into a stenosis site or an occlusion site formed in a blood vessel, a bile duct, a pancreatic duct, or the like and that dilates the stenosis site or the occlusion site to secure flow of blood, bile (biliary fluid), pancreatic juice, or the like.

### 2. Description of the Related Art

When a stenosis site or an occlusion site is formed in a blood vessel, a bile duct, a pancreatic duct, or the like, flow of blood, bile (biliary fluid), pancreatic juice, or the like becomes weak. Hitherto, as a method of treating the stenosis site or the occlusion site, a treatment method using a balloon catheter has been widely used. A balloon catheter mainly includes a balloon that is a dilation body, an outer tube that is secured to the proximal end of the balloon, and an inner tube that is inserted inside the balloon and the outer tube. The inner tube is used to insert a guide wire, and a dilation lumen provided between the outer tube and the inner tube is used for communicating liquid (e.g. a contrast medium or a saline solution) for dilating the balloon. When a balloon catheter is inserted into a blood vessel, a bile duct, a pancreatic duce, or the like, if frictional resistance between the balloon catheter and the blood vessel, the bile duct, the pancreatic duct, or the like is large, the balloon catheter is caught midway and a problem is encountered in that the balloon catheter cannot be inserted into the stenosis site or the occlusion site.

In order to overcome the above problem, a balloon catheter is known that reduces the frictional resistance between the balloon catheter and the blood vessel, the bile duct, the pancreatic duct, or the like by coating a coating agent on the balloon catheter (see Japanese Patent No. 5330506, for example) . In the balloon catheter in Japanese Patent No. 5330506, a coating agent with low concentration and a coating agent with high concentration are coated in multi-layers so as to reduce frictional resistance between the balloon catheter and the blood vessel, the bile duct, the pancreatic duct, or the like and so as to suppress drop in manipulability of the balloon catheter when some of the coating agent comes off during a procedure.

### SUMMARY OF THE INVENTION

However, in the balloon catheter of Japanese Patent No. 5330506, since the coating agent is coated on the entire surface of the balloon, the balloon disadvantageously slips in the front-rear direction when the balloon is dilated in the stenosis site or the occlusion site. Furthermore, although the coating agent is coated in multi-layers, the problem of the coating agent coming off during procedure is not resolved. In particular, when the stenosis site or the occlusion site is formed at the end of the blood vessel, the bile duct, the pancreatic duct, or the like making the procedure time long, the coating agent comes off and the manipulability of the balloon catheter disadvantageously drops. Furthermore, since the coating agent is coated in multi-layers, the process of manufacturing the balloon catheter is lengthy and, as a result, the cost to manufacture the balloon catheter is disadvantageously high.

The present invention has been made in view of the above circumstances and, by forming a rough portion that has a large surface roughness and a smooth portion that has a small surface roughness on the surface of the balloon and by positioning the rough portion between the inner tube and the smooth portion when the balloon is folded, the present invention aims to provide a balloon catheter that reduces frictional resistance between the balloon catheter and the blood vessel, the bile duct, the pancreatic duct, or the like without coating a coating agent and a balloon catheter in which the balloon does not easily slip in the front-rear direction when the balloon is dilated.

The above problems are overcome by the following device.

The present invention refers to a balloon catheter including an inner tube and a balloon secured to the inner tube and foldable around an outer periphery of the inner tube, in which at least one rough portion that has a large surface roughness and at least one smooth portion that has a surface roughness that is smaller than the surface roughness of the rough portion are formed on a surface of the balloon. The at least one rough portion and the at least one smooth portion are configured so that the rough portion is positioned between the inner tube and the smooth portion in a cross sectional view of the balloon catheter, when the balloon is folded around the outer periphery of the inner tube.

I.e., only the smooth portion is externally exposed in the present invention, when the balloon is folded around the outer periphery of the inner tube.

The rough portion may be formed by applying plasma treatment to the surface of the balloon or by physically scrapping the surface of the balloon.

The smooth portion may be formed by pressing the surface of the balloon with a roller that is coated with resin.

In one embodiment, the rough portion and the smooth portion are formed in a circumferential direction around the outer periphery of the inner tube in a dilated state of the balloon.

The surface of the balloon may be formed by a single rough portion and a single smooth portion. Alternatively, the surface of the balloon may be formed by a plurality of rough portions and a plurality of smooth portions.

In the case when the surface of the balloon is formed by the plurality of rough portions and the plurality of smooth portions, the rough portions and the smooth portions may be formed alternately in the circumferential direction around the outer periphery of the inner tube in a dilated state of the balloon. Further, the balloon may be configured to include a plurality of wing portions and a plurality of valley portions positioned between the inner tube and the wing portions in a cross sectional view of the balloon catheter, when the balloon is folded around the outer periphery of the inner tube. The plurality of wing portions may comprise the smooth portions, and the plurality of valley portions may comprise the rough portions.

In the balloon catheter according to the present invention, the rough portion that has a large surface roughness and the smooth portion that has a surface roughness that is smaller than the rough portion are formed on the surface of the balloon, and when the balloon in folded around the outer periphery of the inner tube, the rough portion is positioned between the inner tube and the smooth portion. When a technician inserts the balloon catheter into a blood vessel, a bile duct, a pancreatic duct, or the like, since the smooth portion of the balloon comes in contact with the wall of the blood vessel, the bile duct, the pancreatic duct, or the like, frictional resistance between the balloon catheter and the blood vessel, the bile duct, the pancreatic duct, or the like is reduced and, as a result, the manipulability of the balloon catheter can be improved. Furthermore, when the technician dilates the balloon in the stenosis site or the occlusion site, since the rough portion of the balloon comes in contact with the wall of the blood vessel, the bile duct, the pancreatic duct, or the like, the frictional resistance between the balloon catheter and the wall of the blood vessel, the bile duct, the pancreatic duct, or the like increases and, as a result, the risk of the balloon slipping in the front-rear direction can be reduced. Moreover, since no coating agent is coated, even if the procedure time becomes long, the risk of reduction in manipulability of the balloon catheter that is caused by the coating agent coming off from the balloon does not occur.

### BRIEF DESCRIPTION OF THE DRAWINGS

Fig. 1 is a general view of a balloon catheter (dilated) of an exemplary embodiment;
Fig. 2 is a diagram illustrating a cross-section taken along line II-II of Fig. 1;
Fig. 3 is a general view of a balloon catheter (folded) of the exemplary embodiment;
Fig. 4 is a diagram illustrating a cross-section taken along line IV-IV of Fig. 3;
Fig. 5 is a first modification of the balloon of Fig. 2;
Fig. 6 is a diagram illustrating a folded state of the balloon illustrated in Fig. 5 and is the first modification of the balloon of Fig. 4;
Fig. 7 is a diagram illustrating a folded state of the balloon illustrated in Fig. 5 and is a second modification of the balloon of Fig. 4;
Fig. 8 illustrates a second modification of the balloon of Fig. 2; and
Fig. 9 is a diagram illustrating a folded state of the balloon illustrated in Fig. 8 and is a third modification of the balloon of Fig. 4.

### DESCRIPTION OF THE PREFERRED EMBODIMENTS

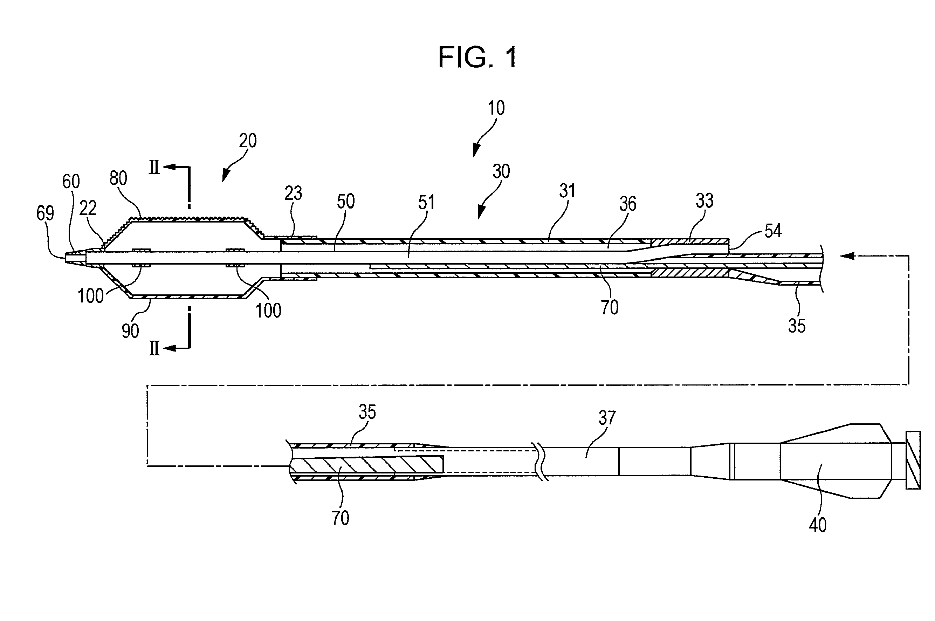

Referring to Figs. 1 to 4, the present invention will be described with an exemplary case in which a balloon catheter 10 of the present exemplary embodiment is used. In Figs. 1 to 3, the left side of the drawing is a distal end side (a distal side) of the balloon catheter 10 which is inserted into the body and the right side of the drawing is a proximal end side (a proximal side or a base end side) of the balloon catheter 10 on which manipulation is carried out by a technician, such as a doctor.

The balloon catheter 10 is used to treat, for example, a stenosis site or an occlusion site that is formed in a blood vessel, a bile duct, a pancreatic duct, or the like. As illustrated in Fig. 1, the balloon catheter 10 mainly includes a balloon 20, an outer tube 30, a connector 40, an inner tube 50, a tip 60, and a reinforcement body 70. Note that Fig. 1 illustrates a dilated state of the balloon 20.

The balloon 20 that dilates a stenosis site or an occlusion site is a member formed of resin and includes a distal end attaching portion 22 on the distal end side and a proximal end attaching portion 23 on the proximal end side. The distal end attaching portion 22 is secured to the distal end of the inner tube 50 and to the tip 60, and the proximal end attaching portion 23 is secured to the distal end of the outer tube 30. In Fig. 1, although the distal end attaching portion 22 is secured to the distal end of the inner tube 50 through the tip 60, the arrangement of the distal end attaching portion 22 is not limited to the above and the distal end attaching portion 22 may be interposed between the distal end of the inner tube 50 and the tip 60. Furthermore, in Fig. 1, although the proximal end attaching portion 23 is secured to an outer periphery of the distal end of the outer tube 30, the arrangement of the proximal end attaching portion 23 is not limited to the above and the proximal end attaching portion 23 may be secured to an inner wall of the distal end of the outer tube 30. The outer tube 30 is a tubular member constituting a dilation lumen 36 for supplying liquid, such as a contrast medium or a saline solution, to dilate the balloon 20. In order from the distal end side, the outer tube 30 includes a distal end outer tube portion 31, a guide wire port portion 33, an intermediate outer tube portion 35, and a proximal end outer tube portion 37. The distal end outer tube portion 31 and the intermediate outer tube portion 35 are tubes formed of resin, such as polyamide, polyamide elastomer, polyolefin, polyester, or polyester elastomer. The guide wire port portion 33 is a portion where the distal end outer tube portion 31, the intermediate outer tube portion 35, and the inner tube 50 are secured. The inner tube 50 is inserted into the distal end outer tube portion 31, and the dilation lumen 36 described above is formed between the distal end outer tube portion 31 and the inner tube 50. The proximal end outer tube portion 37 is a metal tubular member that is a so-called hypo tube. The distal end of the proximal end outer tube portion 37 is inserted into and is secured to the proximal end of the intermediate outer tube portion 35. The connector 40 is attached to the proximal end of the proximal end outer tube portion 37. When liquid, such as a contrast medium or a saline solution, for dilating the balloon 20 is supplied from an indeflator (not shown) that is attachable to the connector 40, the liquid passes through the dilation lumen 36 and dilates the balloon 20. Note that the material of the proximal end outer tube portion 37 is not limited to a particular material and stainless steel (SUS304) or a super-elastic alloy, such as a Ni-Ti alloy, may be used.

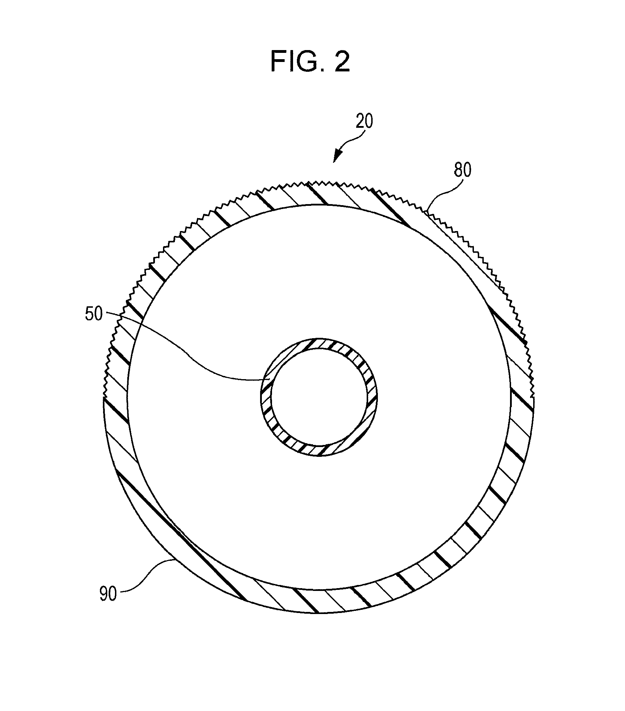

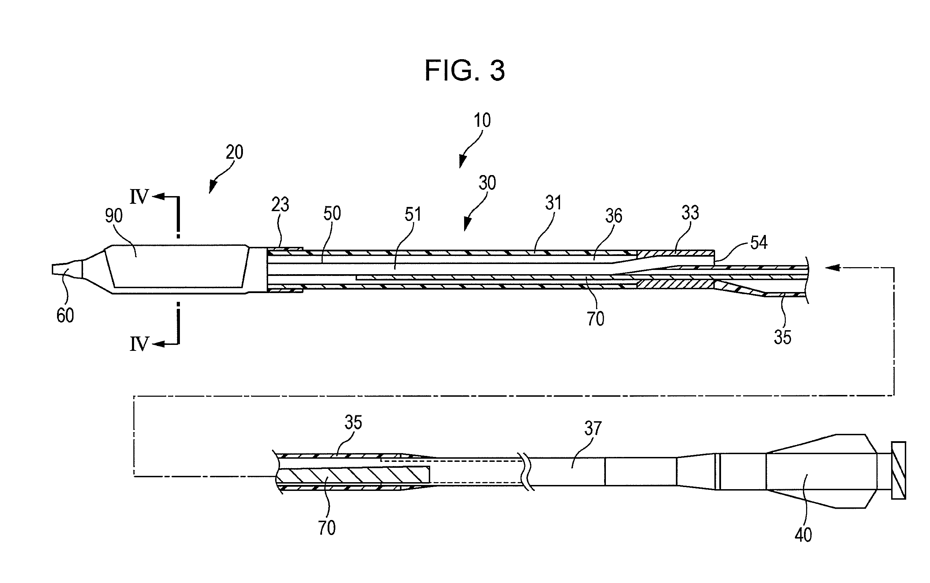

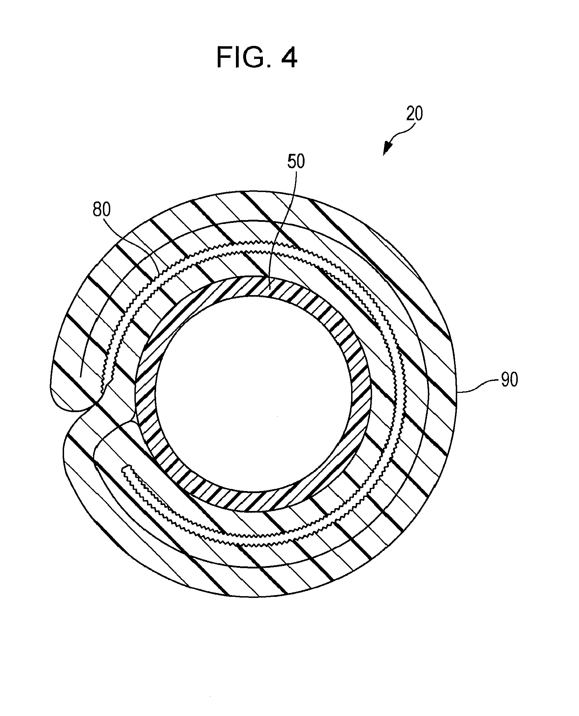

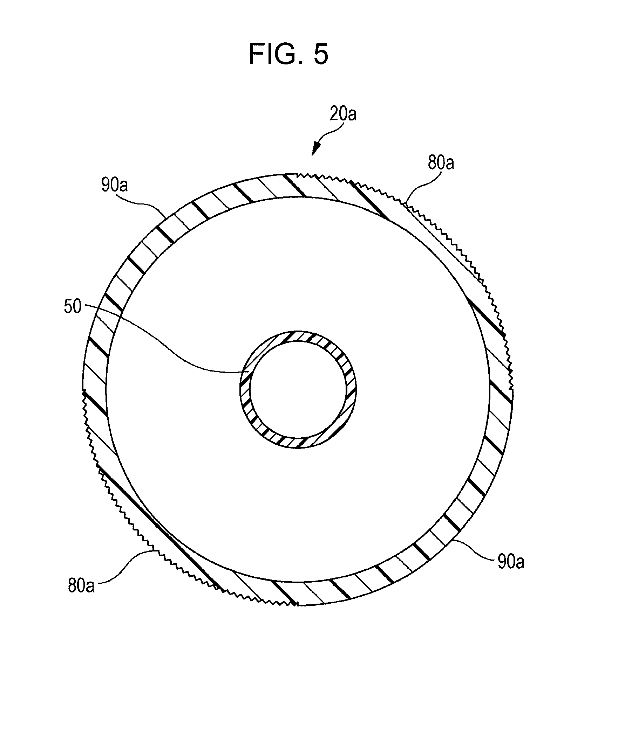

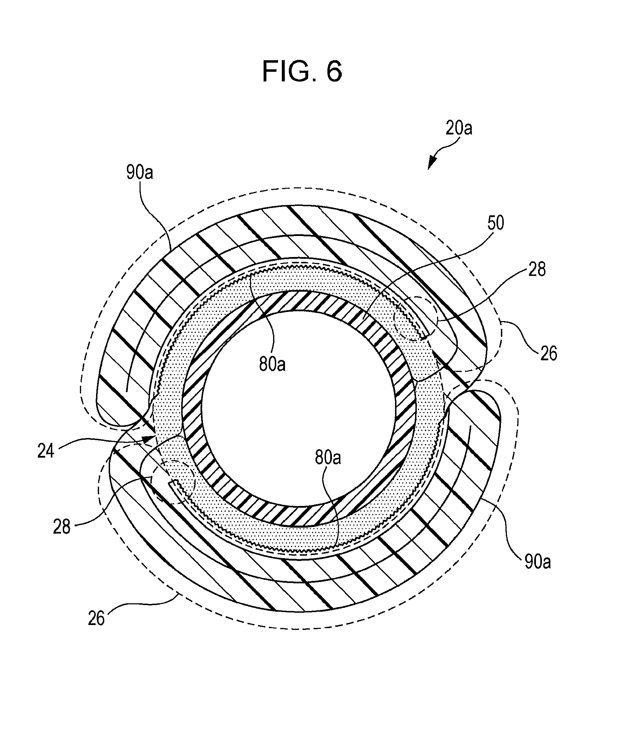

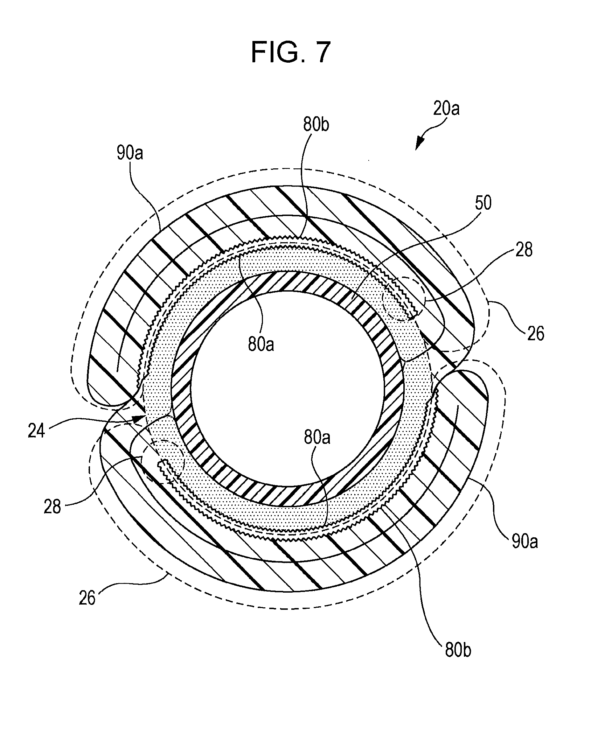

The inner tube 50 forms a guide wire lumen 51 for inserting the guide wire therein. Furthermore, the proximal end of the inner tube 50 is secured to the guide wire port portion 33 of the outer tube 30 so as to form a rear-end-side guide-wire port 54. The distal end of the inner tube 50 is secured to the tip 60 and the distal end attaching portion 22 of the balloon 20. The tip 60 is a member having a tapered outer shape whose outside diameter gradually decreases towards the distal end and is formed of flexible resin. The resin forming the tip 60 is not limited to a particular resin and, for example, polyurethane or polyurethane elastomer may be employed. The tip 60 is a cylindrical member that is secured to the distal end of the guide wire lumen 51 and includes a distal end side guide wire port 69 on the distal end thereof. Two radiopaque markers 100 are attached to the inner tube 50 inside the balloon 20 so as to enable the position of the balloon 20 to be perceived when under radiation exposure. The reinforcement body 70 is attached on the inner circumferential surface of the distal end of the proximal end outer tube portion 37. The reinforcement body 70 has a round cross-section and is a metal wire rod having a tapered shape whose diameter decreases towards the distal end. The material of the reinforcement body 70 is not limited to a particular material and stainless steel (SUS304) or a supraelastic alloy, such as a Ni-Ti alloy, may be used. The reinforcement body 70 passes through the intermediate outer tube portion 35 and the guide wire port portion 33 and extends to the distal end outer tube portion 31. In Fig. 1, although the distal end of the reinforcement body 70 is not fixed to the outer tube 30 and the inner tube 50, the arrangement is not limited to the above arrangement. For example, the distal end of the reinforcement body 70 may be interposed between the outer tube 30 and the inner tube 50 so as to be fixed thereto. A rough portion 80 that has a large surface roughness and a smooth portion 90 that has a surface roughness that is smaller than the rough portion 80 are formed on the surface of the balloon 20. In the rough portion 80, the heights between the protruded portions and the recessed portions are large on average, and in the smooth portion 90, the heights between the protruded portions and the recessed portions are small on average. The method of forming the rough portion 80 and the smooth portion 90 on the surface of the balloon 20 is not limited to a particular method. For example, after fabricating a balloon 20 formed of only the smooth portion, plasma treatment may be applied to the entire balloon 20 that has been partially masked so that the masked portion is formed as the smooth portion 90 and the portion with no masking is formed as the rough portion 80. Alternatively, after fabricating the balloon 20 formed of only the smooth portion, some portions may be physically scraped off with paper or the like such that the portion that has not been scraped is formed as the smooth portion 90 and the physically scraped portion is formed as the rough portion 80. Alternatively, after pre-fabricating a balloon 20 that is formed of only the rough portion 80, by pressing some portion of the balloon 20 with a roller that is coated with resin, the pressed portion may be formed as the smooth portion 90 and the portion that has not been pressed may be formed as the rough portion 80. Fig. 2 illustrates a diagram cut along section II-II of Fig. 1. As illustrated in Fig. 2, a single rough portion 80 that has a large surface roughness and a single smooth portion 90 that has a surface roughness that is smaller than the rough portion 80 are formed on the surface of and in the circumferential direction of the balloon 20. Fig. 3 illustrates a state in which the balloon 20 is folded around the outer periphery of the inner tube 50. Note that different from Fig. 1, in Fig. 3, the portion between the tip 60 and the balloon 20 is illustrated in external view rather than in cross-sectional view for convenience of description. Fig. 4 illustrates a diagram cut along section IV-IV of Fig. 3 (a cross sectional view of the balloon catheter 10). As illustrated in Fig. 4, when the balloon 20 is folded around the outer periphery of the inner tube 50, the rough portion 80 is positioned between the inner tube 50 and the smooth portion 90. When the technician inserts the balloon catheter 10 into a blood vessel, a bile duct, a pancreatic duct, or the like, as illustrated in Fig. 4, only the smooth portion 90 of the balloon 20 comes in contact with the wall of the blood vessel, the bile duct, the pancreatic duct, or the like, and since the rough portion 80 of the balloon 20 is covered by the smooth portion 90, frictional resistance between the balloon catheter 10 and the blood vessel, the bile duct, the pancreatic duct, or the like is reduced and, as a result, the manipulability of the balloon catheter 10 can be improved. Furthermore, when the technician dilates the balloon 20 in the stenosis site or the occlusion site, as illustrated in Fig. 2, not only the smooth portion 90 of the balloon 20, but also the rough portion 80 of the balloon 20 comes in contact with the wall of the blood vessel, the bile duct, the pancreatic duct, or the like; accordingly, the frictional resistance between the balloon catheter 10 and the wall of the blood vessel, the bile duct, the pancreatic duct, or the like increases and, as a result, the risk of the balloon 20 slipping in the front-rear direction can be reduced. Moreover, since there is no need to coat a coating agent on the balloon 20, even if the procedure time becomes long, the risk of reduction in manipulability of the balloon catheter 10 caused by the coating agent coming off from the balloon 20 does not occur. Fig. 5 illustrates a first modification of the balloon 20 of Fig. 2. Rough portions 80a that have a large surface roughness and smooth portions 90a that have a surface roughness that is smaller than the rough portions 80a are each formed alternately on a surface of a balloon 20a in the circumferential direction around the outer periphery of the inner tube 50. As illustrated in Fig. 6, the balloon 20a folded around the outer periphery of the inner tube 50 includes a trunk portion 24 that covers the outer periphery of the inner tube 50, two wing portions 26 that are bent in a fixed direction so as to cover the outer periphery of the trunk portion 24, and two valley portions 28 formed at the roots of the wing portions 26 when the wing portions 26 cover the outer periphery of the trunk portion 24. I.e., the valley portions are positioned between the inner tube 50 and the wing portions 26. Further the wing portions 26 comprise the rough portions 80a and the valley portions 28 comprise the smooth portions 90a. Since the rough portions 80a are formed only on the outer periphery of the trunk portion 24, when the balloon 20a is folded around the outer periphery of the inner tube 50, the rough portions 80a are positioned between the inner tube 50 and the smooth portions 90a and are covered by the wing portions 26 that are formed by the smooth portions 90a. When the technician dilates the balloon 20a in the stenosis site of the occlusion site, the plurality of rough portions 80a of the balloon 20a can come into contact with the wall of the blood vessel, the bile duct, the pancreatic duct, or the like. As illustrated in Fig. 5, since the rough portions 80a of the balloon 20a is in contact with the upper and lower portions of the wall of the blood vessel, the bile duct, the pancreatic duct, or the like, the risk of the balloon 20a slipping in the front-rear direction can be further reduced. Note that in Fig. 6, although the rough portions 80a are only formed on the outer periphery of the trunk portion 24, the arrangement is not limited to the above. As illustrated in Fig. 7, in addition to the rough portions 80a being formed on the outer periphery of the trunk portion 24, rough portions 80b may be formed on the surfaces of the wing portions 26 on the inner tube 50 side.

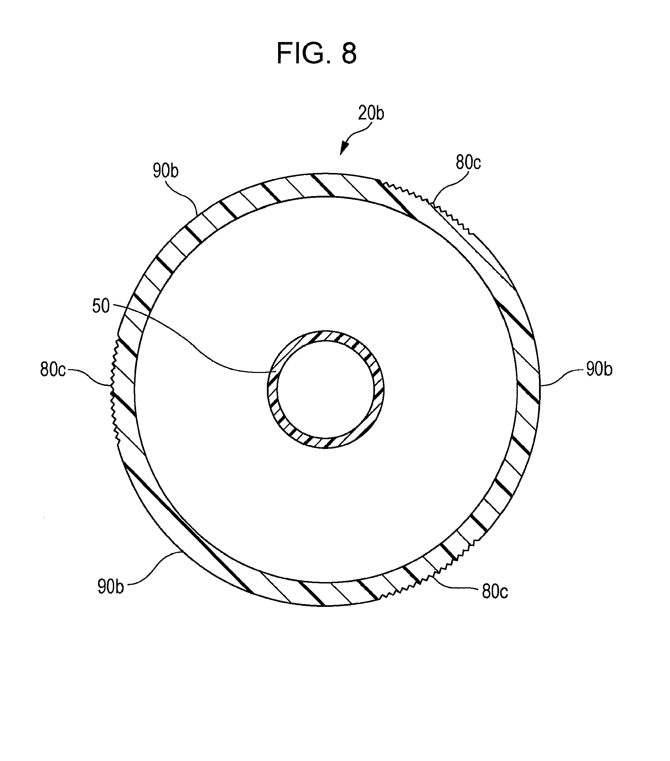

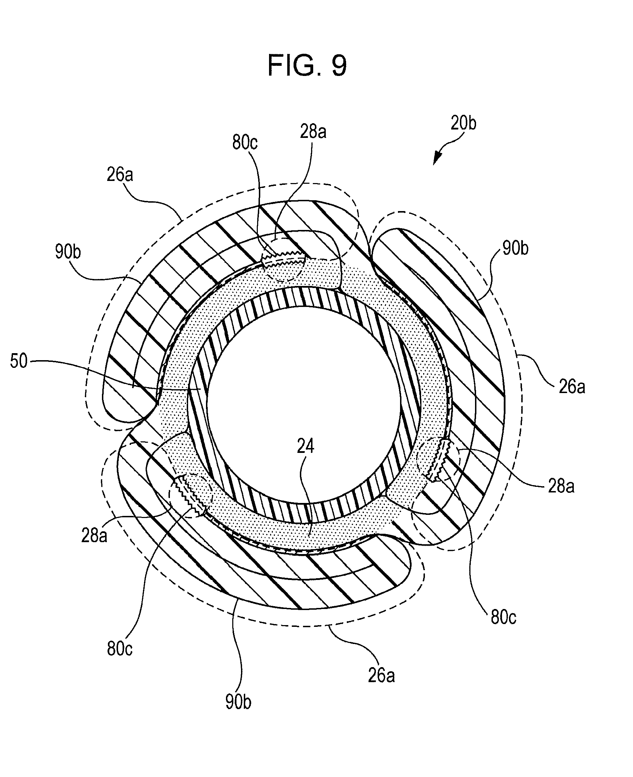

Fig. 8 illustrates a second modification of the balloon 20 of Fig. 2. Three rough portions 80c that have a large surface roughness and three smooth portions 90b that have a surface roughness that is smaller than the rough portions 80c are each formed alternately on a surface of a balloon 20b in the circumferential direction around the outer periphery of the inner tube 50. As illustrated in Fig. 9, the balloon 20b folded around the outer periphery of the inner tube 50 includes a trunk portion 24 that covers the outer periphery of the inner tube 50, three wing portions 26a that are bent in a fixed direction so as to cover the outer periphery of the trunk portion 24, and three valley portions 28a formed at the roots of the wing portions 26a when the wing portions 26a cover the outer periphery of the trunk portion 24. The three wing portions 26a comprise the smooth portions 90b and the three valley portions 28a are formed by the rough portions 80c. Accordingly, when the balloon 20b is folded around the outer periphery of the inner tube 50, the plurality of valley portions 28a that are formed by the rough portions 80c can be easily positioned between the inner tube 50 and the smooth portions 90b.

Furthermore, when the balloon 20b is formed by being heated and compressed inside a mold, a portion of the balloon 20b where a contact pressure with the mold is high becomes to have a large surface roughness and becomes rough, and a portion of the balloon 20b where a contact pressure with the mold is low becomes to have a small surface roughness and becomes smooth. By utilizing the above, by forming the balloon 20b by just performing heating and compression inside the mold, the three valley portions 28a where the contact pressures with the mold are high are formed as the rough portions 80c and the three wing portions 26a where the contact pressures with the mold are low are formed as the smooth portions 90b. Accordingly, the process of forming the rough portions 80c or the smooth portions 90b of the balloon 20b can be excluded. Note that in order to further reduce frictional resistance between the balloon catheter 10 and the wall of the blood vessel, the bile duct, the pancreatic duct, or the like, when the technician inserts the balloon catheter 10 into the blood vessel, the bile duct, the pancreatic duct, or the like, a coating agent may be coated on the outer periphery of the smooth portions 90, 90a, and 90b of the balloons 20, 20a, and 20b, however, it is desirable that no coating agent is coated on the outer periphery of the rough portions 80, 80a, 80b, and 80c of the balloons 20, 20a, and 20b. As described above, when the technician inserts the balloon catheter 10 into the blood vessel, the bile duct, the pancreatic duct, or the like, since only the smooth portion 90, 90a, or 90b of the balloon 20, 20a, or 20b is in contact with the wall of the blood vessel, the bile duct, the pancreatic duct, or the like, frictional resistance between the balloon catheter 10 and the wall of the blood vessel, the bile duct, the pancreatic duct, or the like, can be reduced, and when the technician dilates the balloon 20 inside the blood vessel, the bile duct, the pancreatic duct, or the like, since the rough portions 80, 80a, 80b, or 80c of the balloon 20, 20a, or 20b is in contact with the wall of the blood vessel, the bile duct, the pancreatic duct, or the like, frictional resistance between the balloon catheter 10 and the wall of the blood vessel, the bile duct, the pancreatic duct, or the like, can be increased. Furthermore, since there is no need to coat a coating agent on the balloon 20, 20a, and 20b, even if the procedure time becomes long, the risk of reduction in manipulability of the balloon catheter 10 that is caused by the coating agent coming off from the balloon 20, 20a, and 20b does not occur.

## Claims

1. A balloon catheter (10); comprising:
an inner tube (50), and
a balloon (20, 20a, 20b) secured to the inner tube (50) and foldable around an outer periphery of the inner tube (50), **characterized in that**
at least one rough portion (80, 80a, 80b, 80c) that has a large surface roughness and at least one smooth portion (90, 90a, 90b) that has a surface roughness that is smaller than the surface roughness of the rough portion are formed on a surface of the balloon (20), and
the at least one rough portion (80, 80a, 80b, 80c) and the at least one smooth portion (90, 90a, 90b) are configured so that the rough portion (80, 80a, 80b, 80c) is positioned between the inner tube (50) and the smooth portion (90, 90a, 90b) in a cross sectional view of the balloon catheter (10), when the balloon (20, 20a, 20b) is folded around the outer periphery of the inner tube (50).

2. The balloon catheter (10) according to claim 1, wherein the at least one rough portion (80, 80a, 80b, 80c) is formed by applying plasma treatment to the surface of the balloon (20, 20a, 20b) or by physically scrapping the surface of the balloon (20, 20a, 20b).

3. The balloon catheter (10) according to claim 1 or 2, wherein the at least one smooth portion (90, 90a, 90b) is formed by pressing the surface of the balloon (20, 20a, 20b) with a roller that is coated with resin.

4. The balloon catheter (10) according to any one of claims 1 to 3, wherein
the at least one rough portion (80a, 80c) and the at least one smooth portion (90a, 90b) are formed in a circumferential direction around the outer periphery of the inner tube (50) in a dilated state of the balloon (20, 20a, 20b).

5. The balloon catheter (10) according to any one of claims 1 to 4, wherein
the surface of the balloon (20) is formed by a single rough portion (80) and a single smooth portion (90).

6. The balloon catheter (10) according to any one of claims 1 to 4, wherein
the surface of the balloon (20a, 20b) is formed by a plurality of rough portions (80a, 80b, 80c) and a plurality of smooth portions (90a, 90b).

7. The balloon catheter (10) according to claim 6, wherein
the rough portions (80a, 80c) and the smooth portions (90a, 90b) are formed alternately in the circumferential direction around the outer periphery of the inner tube (50) in a dilated state of the balloon (20a, 20b).

8. The balloon catheter (10) according to claim 6 or 7, wherein
the balloon (20a, 20b) is configured to include a plurality of wing portions (26, 26a) and a plurality of valley portions positioned between the inner tube (50) and the wing portions (26, 26a) in a cross sectional view of the balloon catheter (10), when the balloon (20a, 20b) is folded around the outer periphery of the inner tube (50), (28, 28a),
the plurality of wing portions comprise the smooth portions (90a 90b), and
the plurality of valley portions comprise the rough portions (80a, 80b, 80c).
